# EUROPEAN PATENT APPLICATION

(11) **EP 4 792 832 A1**
(43) Date of publication of application: **19.08.2026**
(21) Application number: 25203167.9
(22) Date of filing: 18.09.2025
(51) Int. Cl.: A61K 41/00, A61K 47/64, A61P 35/00, C07K 7/50, C07F 5/02

(54) **PEPTIDE-BOUND BORON CLUSTER COMPLEX FOR USE IN BORON NEUTRON CAPTURE THERAPY (BNCT)**

(30) Priority: 17.02.2025 EP 25382134
(71) Applicant: Labs in Love, S.L., 28002 Madrid (ES); Consejo Superior de Investigaciones Científicas (CSIC), 28006 Madrid (ES); Universidad de Granada, 18071 Granada (ES)
(72) Inventor: Nuez Martínez, Miquel, 08191 Barcelona (ES); Porras Quesada, María Isabel, 18008 Granada (ES); Núñez Aguilera, Rosario, 08290 Cerdanyola del Vallés (ES); Porras Sánchez, José Ignacio, 18008 Granada (ES); Ruiz Ruiz, María Carmen, 18008 Granada (ES)
(74) Representative: Clarke, Modet y Cía., S.L.

(57) **Abstract**

The present invention relates to a peptide-bound boron cluster complex for use in boron neutron capture therapy (BNCT). The present invention covers a library of complexes where one or more icosahedral boron clusters, such as carboranes and metallacarboranes, are linked to peptides or hormone analogues that bind to receptors or antigens overexpressed in tumors.

## Description

### TECHNICAL FIELD

The invention described herein relates to the field of boron neutron capture therapy (BNCT). Specifically, the invention relates to a peptide-bound boron cluster complex based on icosahedral boron clusters for use in boron neutron capture therapy (BNCT).

### BACKGROUND OF THE INVENTION

Cancer is the second cause of death in developed countries and is expected to become the first by 2030. With more than 200 variants, each requiring different diagnostic and therapeutic approaches, the disease is a major challenge.

One of the most promising cancer therapies today is Boron Neutron Capture Therapy (BNCT). BNCT exploits the ability of stable boron-10 (¹⁰B) isotopes to absorb low-energy neutrons and suffering disintegration, emitting radiation in the process.

This emitted radiation has a range of a few microns, smaller than the size of a cell. Ideally, ¹⁰B-enriched drugs that selectively target tumor tissue could deliver BNCT without damaging healthy cells. As the only external beam radiotherapy that is selective at the cellular level, it holds great promise for cancers that are diffuse or infiltrate critical organs, which are difficult to treat with current radiotherapies.

BNCT has been used in certain poor-prognosis tumors that have not responded to conventional treatments, in particular brain tumors such as glioblastoma and recurrent head and neck cancers for which current therapies are not effective.

Clinically approved drugs for BNCT include ¹⁰B-enriched boronophenylalanine and sodium borocaptate, the latter currently inactive due to low selectivity. Although ¹⁰B-enriched boronophenylalanine shows some selectivity due to overexpression of the amino acid transporter in tumour cells, its boron content remains low as it carries only one boron atom per molecule. In addition, it shows solubility problems and must be prepared in a fructose solution just at the time of administration. Moreover, the boron content entered in the tumor cells is observed also to escape from cells with time.

Developing new boron carriers with improved selectivity and increased boron loading would advance this technique and promote its use in the clinic as a first-line cancer treatment. Peptide-binding cell surface receptors are typically overexpressed on cancer cells. Therefore, they are interesting targets for targeted tumour therapy. One way to get around these problems is to attach boron-containing compounds to peptide conjugates that specifically target tumour cells through their overexpressed receptors [1].

Different ways (by studying different syntheses or different linkers) of linking these boron-containing compounds to peptides have been studied for years.

Schirrmacher, E., et al. (2003) describes a closo-o-carborane conjugated Tyr₃-octreotate (TATE) derivative as a potential compound for BNCT obtained via Fmoc solid phase peptide synthesis. The boron cluster [C₂B₁₀H₁₁] was introduced through the reaction of 6,9-bis(acetonitrile)decaborane and 5-hexynoic acid yielding a closo-o-carborane conjugated carboxylic acid which was coupled subsequently with solid phase conjugated Tyr₃-octreotate. The compound obtained was considered as a potential compound for BNCT [2].

Betzel, T., et al. (2008) discloses complexes formed by coupling *closo*-*o*-carborane clusters with Tyr₃-octreotatepeptide derivatives (TATE). The complexes could be used in cancer therapy by BNCT. The synthetic peptide Tyr₃-octreotate (TATE) was chosen as a high-affinity and internalizing tumor targeting vector (TTV). Boron cluster compounds, containing 10 or 20 boron atoms, were coupled to the N-terminus of TATE. The obtained affinity data demonstrate that the use of a spacer between TATE and the closo-o-carborane moiety is the option to avoid a loss of biological affinity of closo-o-carborane conjugated TATE. For the first time, it was shown that closo-o-carborane conjugated regulatory peptides retain high biological affinity and selectivity toward their transmembrane tumor receptors [3].

Kawai, K., et al. (2020) illustrates a cyclic RGD (cRGD) peptide-conjugated boronated albumin to direct toward integrin αvβ3, which overexpresses on many cancer cells. A stepwise conjugation of c[RGDfK(Mal)] and maleimide-conjugated *closo*-dodecaborate ([B₁₂H₁₂]²⁻) (MID) to bovine serum albumin (BSA) afforded cRGD-MID-BSA. An in vivo BNCT study revealed that the cRGD peptide ligand combination enhanced accumulation of MID-BSA into tumor cells [5].

US2020114013A1 relates to a boron enriched linker compositions and methods of making boron enriched linkers. The boron enriched linkers can be conjugated to antibodies or antibody fragments to create Antibody Boron Conjugates ("ABCs") to provide a method of treating cancer, immunological disorders and other disease by utilizing BNCT.

Mishiro, K., et al. (2022) evaluate a probe of *closo*-dodecaborate-(Ga-DOTA)-c(RGDfK) (Ga= gallium, DOTA= 1,4,7,10-tetraazacyclododecane-1,4,7,10-tetraacetic acid, and c(RGDfK)= cyclo(arginine-glycine-aspartate-d-phenylalanine-lysine)) containing *closo*-dodecaborate ([B₁₂H₁₂]²⁻) as a boron cluster for BNCT [6].

However, many problems remain in terms of peptide conjugation or binding, the availability of boron in these structures and the efficacy of BNCT in the treatment of a variety of cancers.

There is therefore a need to find new compounds consisting of peptide-bound boron clusters that are stable and effective for use in BNCT.

### DESCRIPTION OF THE INVENTION

The objective of the present invention is to provide high-boron-content compounds based on peptides and hormone analogues for BNCT.

The inventors of the present invention, after extensive and exhaustive research, have surprisingly found an alternative complex based on icosahedral boron clusters, such as carboranes and metallacarboranes, linked to peptides for use in BNCT.

The conjugation of the peptides with icosahedral boron clusters has two aims: to improve drug selectivity through targeting receptors that are over-expressed in different tumors, and to increase the concentration of boron in tumors to improve the efficacy of therapy, potentially reducing the dose to the patient and enabling multimodal therapy.

The present invention covers a library of compounds where one or more icosahedral boron clusters are linked to peptides or hormone analogues that bind to receptors or antigens overexpressed in tumors. By opening a dioxanate ring covalently linked to the boron cluster, the terminal chemical group (-COOH, -COO⁻, -NH₂, or -NH₃⁺) allows for attachment to the corresponding peptide or hormone analogue via an organic chain, either through standard chemical procedures or Solid Phase Peptide Synthesis (SPPS), being joined through a peptide bond (CONH). While similar compounds have been synthesized previously using click-type reactions to link clusters with peptides or hormone analogues, the present invention explores additional enhancements, such as incorporating more amino acids or organic chains to further separate the boron cluster from the analog.

Therefore, the present invention solves the technical problems present in the state of the art and mentioned above.

In a first aspect, the present invention refers to a peptide-bound boron cluster complex characterized in that it has a chemical structure according to one of the following formulas: 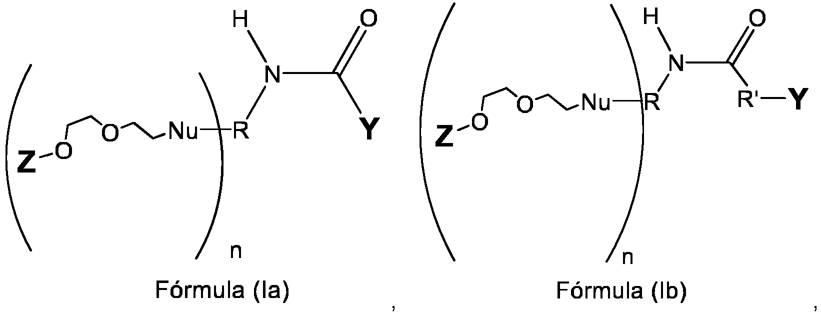 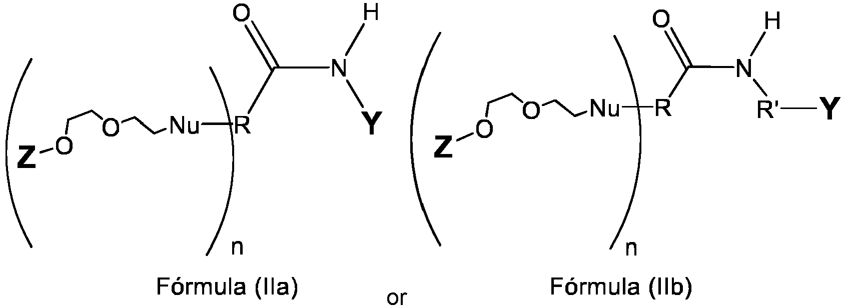 wherein
Z is an icosahedral boron compound selected from the group consisting of closo-dodecaborate, carboranes or isomers thereof, or metallacarboranes;
Nu is a nucleophile selected from the group consisting of halides, thioethers, amines, alkoxides, and carboxylates or derivatives thereof;
R is an aliphatic organic chain, an aromatic organic chain or a polyether chain;
R' is a peptide formed with α-amino acids, β-amino acids, γ-amino acids and/or molecules with at least one -COO⁻ group and/or with at least one -NH₂ group linked together by peptide bonds;
Y is a peptide selected from the group consisting of somatostatin analog peptide, prostate-specific membrane antigen (PSMA) ligand, neuropeptide Y, arginylglycylaspartic acid (RGD) peptides, GE11 peptide, angiopep-2 peptide, gastrin-releasing peptide receptor (GRPR) analogs, luteinizing-hormone-releasing hormone (LHRH) analogue, pseudo-neurotensin derivatives or Lyp-1 peptide, chlorotoxine and apicoline analog or
Y is an anti-solid tumor antibody or a fragment thereof;
n is between 1 and 8;
and its pharmaceutically acceptable salts, solvates hydrates or mixtures thereof.

In a preferred embodiment, the icosahedral boron compound is selected from the group consisting of *closo* [B₁₂H₁₂]²⁻, closo [CB₁₁H₁₂]⁻, isomers of closo C₂B₁₀H₁₂ carboranes (*ortho*-(1,2), *meta-*(1,7), and *para*-(1,12)), isomers of *nido* [C₂B₁₀H₁₁]⁻ carboranes (*ortho*-(1,2) and *meta*-(1,7)) and metallacarboranes [3,3'-M(1,2-C₂B₉H₁₁)₂]⁻, wherein M is a metal selected from Co (III) and Fe(III).

In an embodiment, Y is a peptide which can be selected from a somatostatin analog peptide such asTyr3-octreotate, Tyr3-octreotide a PSMA ligand such as (S)-2-(3-((S)-5-Amino-1-carboxypentyl)ureido)pentanedioic Acid, neuropeptide Y, RGD peptides such as RGD, cyclic RGD, iRGD, GE11 peptide, angiopep-2 peptide, -GRPR analogs, luteinizing-hormone-releasing hormone (LHRH) analogue such as[D-Lys6]-GnRH-I, pseudo-neurotensin derivatives such as [Lys8-9]NT(8-13) or Lyp-1 peptide, chlorotoxine and apicoline analog peptides such as MiniCTX3 and miniAp-4. This list does not represent any limitation.

In another embodiment, Y is an anti-solid tumor antibody which can be selected from anti-nectin-4, anti-EGFR and anti-TROP2.

In a preferred embodiment, the complex of the present invention has the following chemical structure:

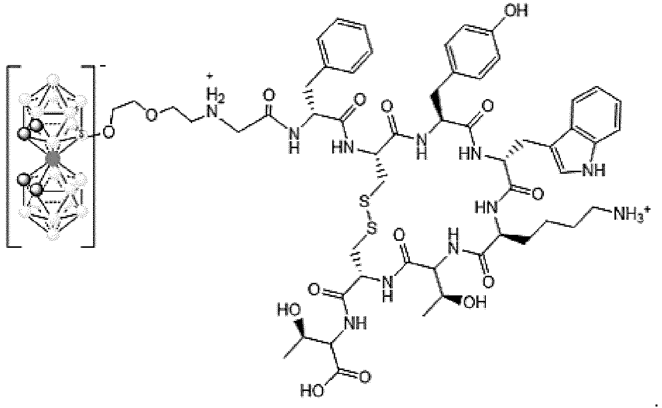

In another preferred embodiment, the complex of the present invention has the following chemical structure:

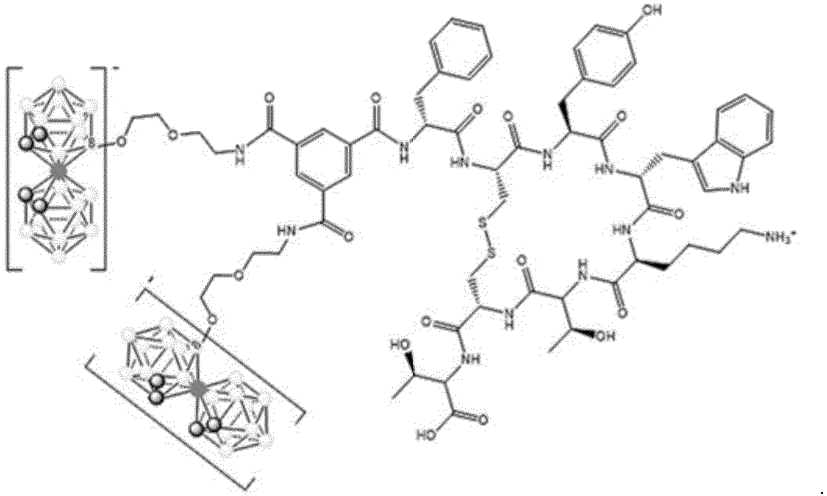

In another preferred embodiment, the complex of the present invention has the following chemical structure:

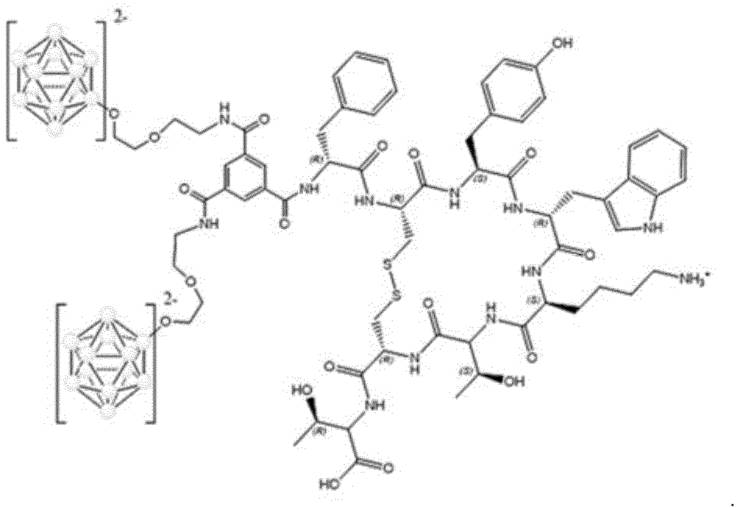

In another preferred embodiment, the complex of the present invention has the following chemical structure:

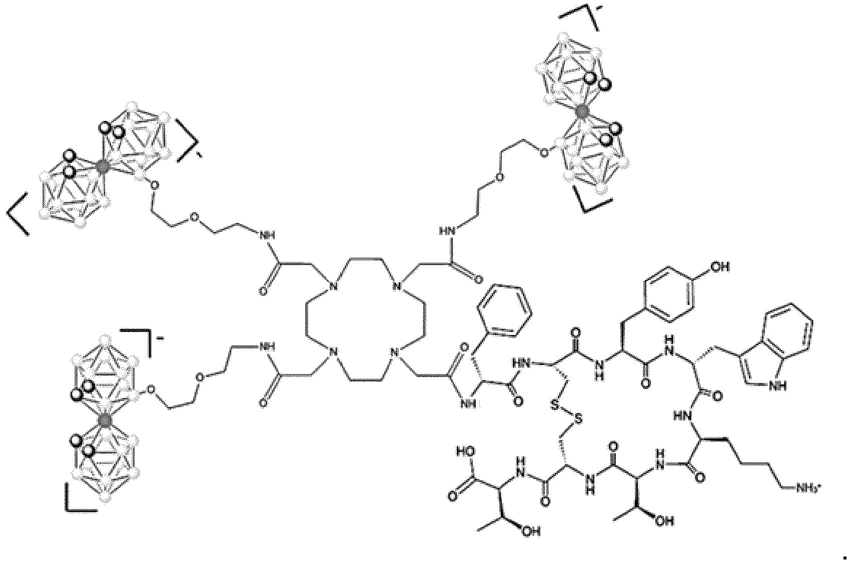

In one embodiment, R has at least one macrocyclic chelator attached for coordination with a radioisotope. This embodiment can be used for imaging techniques such as magnetic resonance imaging (MRI), SPECT or PET. Preferably, the radioisotope is selected from the list consisting of technetium-99m, gallium-68, lutetium-177, terbium isotopes or an atom such as gadolinium.

In another embodiment, at least one B-H vertex of the icosahedral boron compound is replaced by B-X wherein X is a halogen. Preferably the halogen is iodine, and it can serve as a contrast agent for PET and SPECT or as a photosensitiser for radiotherapy.

In a second aspect, the present invention refers to a pharmaceutical composition comprising the peptide-bound boron cluster complex and a pharmaceutically acceptable excipient.

### BRIEF DESCRIPTION OF DRAWINGS

The following figures are described below. These illustrate the exemplary embodiments and are not limiting their scope.
Figure 1 shows the scheme of synthesis of COSAN-TATE.
Figure 2 shows the scheme of synthesis of COSAN₂-TATE.
Figure 3 shows the scheme of synthesis of Dodeca₂-TATE.
Figure 4 shows the scheme of synthesis of COSAN₃-DOTA-TATE.
Figure 5 shows the survival fraction of tumor cells as a function of the neutron dose treated with BPA and COSAN-TATE.

### EXAMPLES

### Example 1. Synthesis of peptide-bound boron cluster complex COSAN-TATE, via solid phase peptide synthesis (SPPS)

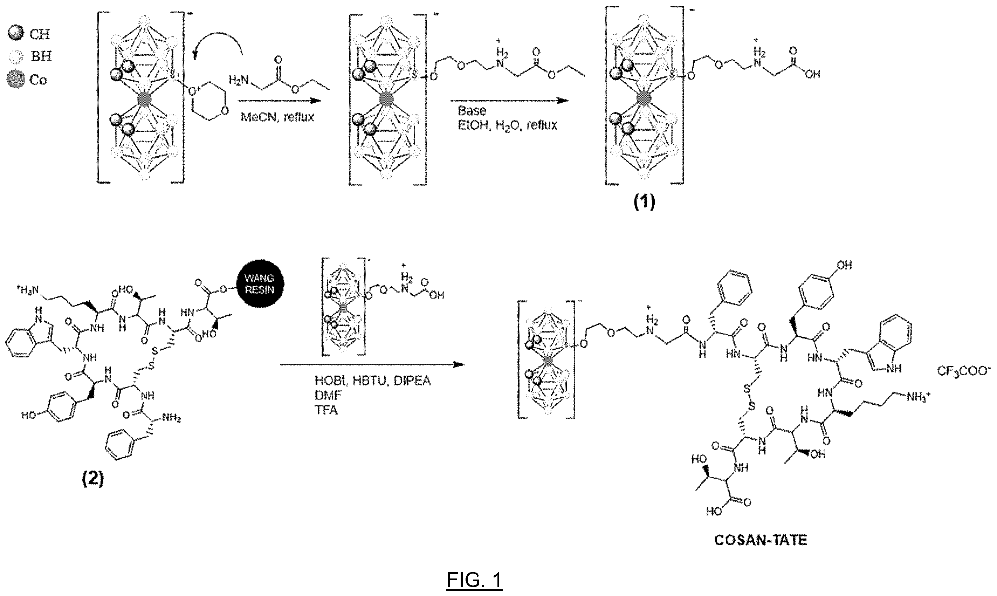

The first step is the synthesis of the boron cluster ligand H[COSAN-bisethylenedioxo-glycine-COOH] (1) (H[3,3'-Co(8-(C₄H₈O₂NHCH₂COOH)-1,2-C₂B₉H₁₀)(1',2'-C₂B₉H₁₁)]) (Fig. 1): COSAN-dioxanate (661 mg, 1,44 mmol) and deprotonated ethyl glycinate (331 mg, 2,88 mmol) were refluxed in 70 mL of dry MeCN for 6h in inert conditions (N₂). COSAN-bisethylenedioxo-glycine ethyl ester was obtained and purified by column chromatography using DCM:Acetone 3:2 as eluent, Rf: 0,58, 743,3 mg. The yield obtained was 89,9%.

H[COSAN-bisethylenedioxo-glycine ethyl ester] (600 mg, 1,17 mmol) was dissolved in 20 mL of EtOH; aqueous NaOH (4015 mg; 100,62 mmol in 80 mL of distilled water) was added to the solution and refluxed for 8h. After the reaction, 60 mL of distilled water were added and brought to acidic pH (using 3M HCl), leading to the complete precipitation of a dark orange solid. The aqueous phase is extracted using DCM (3x 25 mL) and finally all organic phases together were dried with MgSO₄ and evaporated to obtain compound 1 as a dark orange solid (542,2 mg). The yield obtained was 95,6%.

The second step is the reaction of compound 1 with resin supported Tyr₃-octreotate (TATE), (synthesized as in ref. [3]) via SPPS (Fig. 1): 0,4 mmol of 1 (4 equivalents) and 1-Hydroxybenzotriazole hydrate (HOBt-hydrate) (0,4 mmol, 4 equivalents) were dissolved in DMF (1 mL). Hexafluorophosphate benzotriazole tetramethyl uronium (HBTU) (0,4 mmol, 4 equivalents) was added, and the solution was placed in an ultrasonic bath for 5 minutes or complete dilution of the solids. Diisopropylethylamine (DIPEA) (0,8 mmol, 8 equivalents) was then added to the solution, which was vortexed, incubated for 1 minute, and added to 0,1 mmol of N-R-Fmoc-O-tBu-threonine Wang resin with protected Tyr3-octreotate loaded in it (Fig. 1). The mixture was shaken for 4 hours, after which the reaction mixture was removed, and the resin washed with DMF (6 × 1 mL).

To cleave the compound from the resin it was added to a round bottomed flask and treated with a cleavage solution (100 µL/mg resin; 95% TFA, 2,5% H₂O, 2,5% TIS) for 1,5 hours at room temperature and agitation. The mixture was then filtered through a glass frit, and the remaining solid was washed with TFA (4 mL). The filtrate was evaporated under reduced pressure, cold Et₂O (~10 mL) and the crude scratched in order to obtain a crystalline solid/powder which was vacuum filtered, cleaned with Et₂O and petroleum ether and air dryed). The resulting compound, named COSAN-TATE was obtained as a solid (97,6 mg). The yield obtained was 63%. The Formula of the complex is C₆₂H₉₇O₁₇N₁₁S₂F₃B₁₈Co.

The COSAN-TATE synthesis scheme is shown in Figure1.

### Example 2. Synthesis of peptide-bound boron cluster complex COSAN₂-TATE, via solid phase peptide synthesis (SPPS)

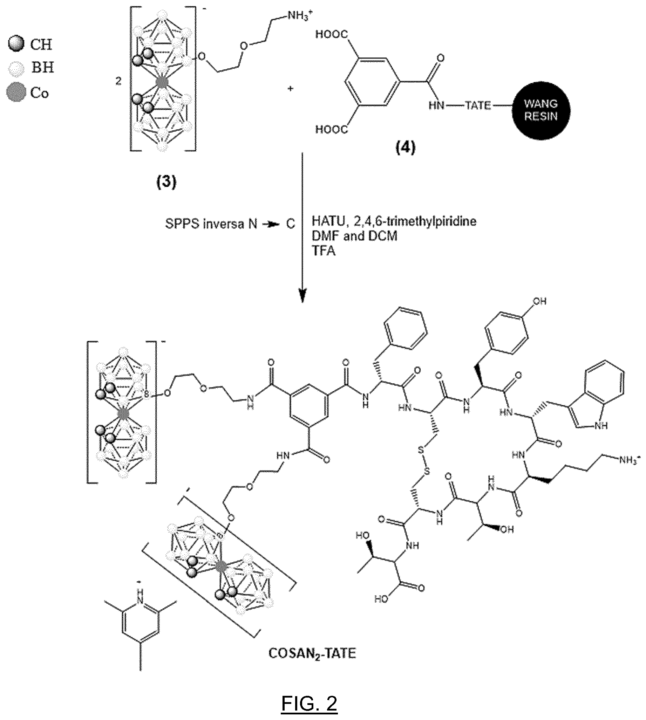

The first step is the synthesis of the boron cluster linker H[COSAN-bisethylenedioxo-NH₂] (H[3,3'-Co(8-(C₄HsO₂NH₂)-1,2-C₂B₉H₁₀)(1',2'-C₂B₉H₁₁)]) (3) (Fig. 2): COSAN-dioxanate (1000 mg, 2,43 mmol) was dissolved in 28 mL of EtOH and 30% aq. NH₃ was added dropwise (9,72 mL, 168 mmol). The mixture was refluxed for 12 h. After cooling, 20 mL of distilled water were added to the crude and the solution was partially evaporated until the complete precipitation of an orange solid, which was vacuum filtered and cleaned with water and petroleum ether to obtain 868,1 mg of (3) as an orange solid. The yield obtained was 83%. This yield is higher than the yield of 70% reported in the literature [7].

Alternatively, the inventors of the present invention has developed a more efficient and straightforward synthesis directly from Cs[COSAN] (Cs[3,3'-Co(1',2'-C₂B₉H₁₁)₂] for the first time, avoiding the purification steps to obtain pure COSAN-dioxanate . The first step of this reaction was carried out in inert conditions: 20 mL of dry 1,4-dioxane were added to 350 mg (0,766 mmol, 1 equivalent)) of Cs[COSAN] and then 0,95 mL of BF₃·Et₂O (7,66 mmol; 10 eq.) were added. After 5h of refluxing, 7 mL of NH₃ (30% in aqueous solution) (120,96 mmol; 158 eq) were added. The reaction was refluxed overnight. After cooling the reaction, the white precipitate was filtered off and the solution evaporated to give an orange residue. A minimum amount of EtOH was added to dissolve the orange solid, while white solids are filtrated and discarded. 10 mL of distilled water were added to the EtOH, and the solution was evaporated (bath max. temperature: 40°C) until an orange precipitate appears. This precipitate was filtered and washed with water and petroleum ether obtaining 220 mg of product. The yield obtained was 67%.

To compare, the synthesis of COSAN-dioxanate has a yield of 57%, meaning that the global process to obtain H[COSAN-bisethylenedioxo-NH₂] has a yield of 47%. With this new procedure, the process is shorter, and the yield has improved by 20%.

The second step is the addition of trimesic acid to the resin supported Tyr₃-octreotate via SPPS to give resin (4) (Fig. 2): Trimesic acid (0,4 mmol, 4 equivalents) and O-(7-azabenzotriazol-1-yl)-N,N,N',N'-tetramethyluronium hexafluorophosphate (HATU) (0,4 mmol, 4 equivalents) were dissolved in 1 ml of N-Methyl-2-pyrrolidone, and 734 µl of 0,6 M 1-Hydroxy-7-azabenzotriazole (HOAt) in DMF (0,44 mmol, 4,4 equivalents) were added to the solution. The solids are dissolved using an ultrasonic bath if needed. 105 µl of DIPEA (0,6 mmol, 6 equivalents) are added to the solution which was vortexed, incubated for 1 minute, and added 0,1 mmol of N-R-Fmoc-O-tBu-threonine Wang resin with protected Tyr3-octreotate loaded in it. The mixture was shaken for 20 minutes, after which the reaction mixture was removed, and the resin washed with DMF (6 × 1 mL) obtaining 0,1 mmol of N-R-Fmoc-O-tBu-threonine Wang resin with protected Tyr3-octreotate linked to trimesic acid loaded in it (4).

The third step is the addition of two linkers of (3) to trimesic acid terminal of Tyr₃-octreotate (4) via inverse SPPS (see Figure 2). 1 mmol of (3) (10 eq., 5 eq. per -COOH terminal) was dissolved in 3,8 mL of DCM and added to the resin 4, next, a solution of HATU (0,8 mmol, 8 eq., 4 eq. per -COOH terminal) in 950 µl of DMF was also added to the resin. Finally, 236 µl of 2,4,6-trimethylpyridine (1,8 mmol, 18 eq., 9 eq. per -COOH terminal) were added to 0,1 mmol of 4, and the reaction time extended for 5 hours, after which the reaction mixture was removed and the resin washed with DMF (6 × 1 mL).

To cleave the compound from the resin it was added to a round bottomed flask and treated with a cleavage solution (100 µL/mg resin; 95% TFA, 2,5% H₂O, 2,5% TIS) for 1,5 hours at room temperature and agitation. The mixture was then filtered through a glass frit, and the remaining solid was washed with TFA (4 mL). The filtrate was evaporated under reduced pressure, cold Et₂O (~10 mL) and the crude scratched to obtain a crystalline solid/powder which was vacuum filtered, cleaned with Et₂O and petroleum ether and air dryed 121,2 mg COSAN₂-TATE were obtained. The yield obtained was 56%. The Formula of the complex is C₈₃H₁₄₀O₁₇N₁₃S₂B₃₅CO₂.

The COSAN₂-TATE synthesis scheme is shown in Fig. 2.

### Example 3. Synthesis of peptide-bound boron cluster complex Dodeca₂-TATE, via solid phase peptide synthesis (SPPS)

The first step is the synthesis of the boron cluster linker N(*n*Bu)₄H[B₁₂H₁₁-bisethylenedioxo-NH₂] (5) or (N(*n*Bu)₄H[1-(C₄H₈O₂NH₂)B₁₂H₁₁]): N(*n*Bu₄)[B₁₂H₁₁-dioxanate] (750 mg, 1,63 mmol) was dissolved in 22,5 mL of EtOH and 30% aq. NH₃ was added dropwise (7,5 mL, 129,6 mmol). The mixture was refluxed for 15,5 h. After cooling, 25 mL of distilled water were added to the crude and some white solid precipitated, the solution was partially evaporated until the complete precipitation of a white solid, which was vacuum filtered and cleaned with water and petroleum ether to obtain 591,6 mg of (5) as a white solid. The yield obtained was 76%.

The second step is the addition of trimesic acid to resin supported Tyr₃-octreotate via SPPS, as in example 2.

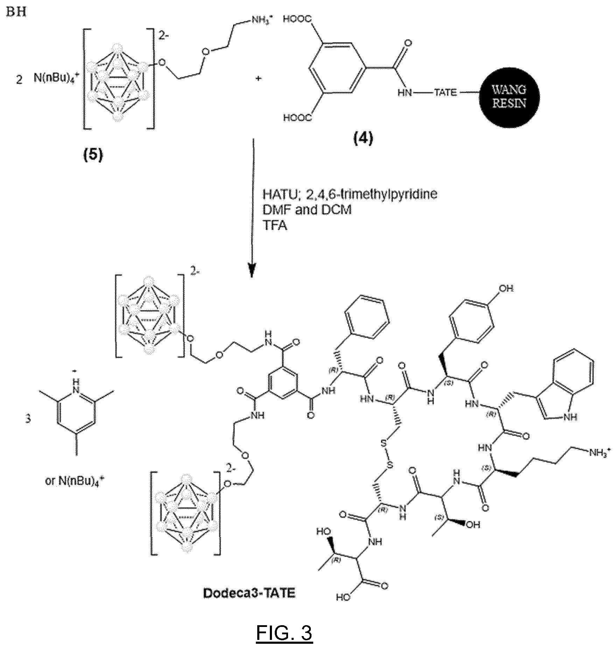

The third step is the addition of two linkers of (5) to trimesic acid terminal of Tyr₃-octreotate (4) via inverse SPPS (Fig. 3): 1 mmol of (5) (10 eq., 5 eq. per -COOH terminal) was dissolved in 3.8 mL of DCM and added to the resin (4), next, a solution of HATU (0,8 mmol, 8 eq., 4 eq. per - COOH terminal) in 950 µl of DMF was also added to the resin. Finally, 236 µl of 2,4,6-trimethylpyridine (1.8 mmol, 18 eq., 9 eq. per -COOH terminal) were added to 0,1 mmol of (4), and the reaction time extended for 19 hours, after which the reaction mixture was removed and the resin washed with DMF (6×1mL).

To cleave the compound from the resin it was added to a round bottomed flask and treated with a cleavage solution (100 µL/mg resin; 95% TFA, 2,5% H₂O, 2,5% TIS) for 1,5 hours at room temperature and agitation. The mixture was then filtered through a glass frit, and the remaining solid was washed with TFA (4 mL). The filtrate was evaporated under reduced pressure, cold Et₂O (~10 mL) and the crude scratched to obtain a crystalline solid/powder which was vacuum filtered, cleaned with Et₂O and petroleum ether and air dryed).

22,4 mg of solid (the complex) were obtained with a yield of 11,9%. The Formula of the complex is C₈₂H₁₄₃N₁₅O₁₅S₂B₂₄.

The Dodeca₂-TATE synthesis scheme is shown in Figure 3.

### Example 4. Synthesis of peptide-bound boron cluster complex COSAN₃-DOTA-TATE, via solid phase peptide synthesis (SPPS)

The first step is the synthesis of (3) described previously. The second step is the addition of DOTA to resin supported Tyr₃-octreotate via SPPS to obtain (6): DOTA (0,4 mmol, 4 equivalents) and HATU (0,4 mmol, 4 equivalents) were dissolved in 1 ml of N-Methyl-2-pyrrolidone, and 734 µl of 0,6 M HOAt in DMF (0,44 mmol, 4,4 equivalents) were added to the solution. The solids are dissolved using an ultrasonic bath if needed. 105 µl of DIPEA (2,2 mmol, 22 equivalents) are added to the solution, which was vortexed, incubated for 1 minute, and added 0,1 mmol of N-R-Fmoc-O-tBu-threonine Wang resin with protected Tyr3-octreotate loaded in it. The mixture was shaken for 18,5h hour, after that, the reaction mixture was removed and the resin washed with DMF (6 × 1 mL) obtaining 0,1 mmol of N-R-Fmoc-O-tBu-threonine Wang resin with protected Tyr3-octreotate linked to DOTA loaded in it (6).

The third step is the addition of three linkers of (3) to DOTA acid terminals of Tyr₃-octreotate (6) via inverse SPPS: 1,5 mmol of (3) (15 eq., 5 eq. per -COOH terminal) was dissolved in 5,7 mL of DCM and added to the resin (6), next, a solution of HATU (1,2 mmol, 12 eq., 4 eq. per -COOH terminal) in 1425 µl of DMF was also added to the resin. Finally, 354 µl of 2,4,6-trimethylpyridine (2,7 mmol, 27 eq., 9 eq. per -COOH terminal) were added to 0.1 mmol of (6), and the reaction time extended overnight, after which the reaction mixture was removed and the resin washed with DMF (6×1mL).

To cleave the compound from the resin it was added to a round bottomed flask and treated with a cleavage solution (100 µL/mg resin; 95% TFA, 2,5% H₂O, 2,5% TIS) for 1,5 hours at room temperature and agitation. The mixture was then filtered through a glass frit, and the remaining solid was washed with TFA (4 mL). The filtrate was evaporated under reduced pressure, cold Et₂O (~10 mL) and the crude scratched to obtain a crystalline solid/powder which was vacuum filtered, cleaned with Et₂O and petroleum ether and air dryed).

The resulting complex was named COSAN₃-DOTA-TATE. 56,2 mg of solid (the complex) were obtained with a yield of 20%. The Formula of the complex is C₁₀₅H₂₀₀N₁₂O₂₂CO₃S₂B₅₄.

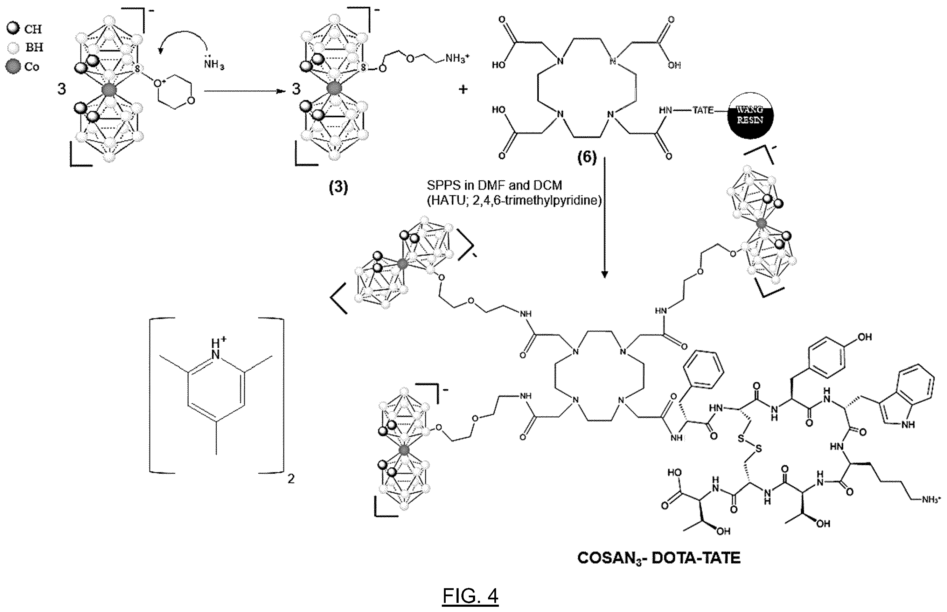

The COSAN₃-DOTA-TATE synthesis scheme is shown in Figure 4.

### Example 5. Biological tests of COSAN-TATE

The complex COSAN-TATE synthetized in example 1 was used to test if the addition of the boron cluster to the peptide do not impair its ability to attach the receptors. For this purpose, the boron uptake of tumor cells overexpressing the somatostatine receptor with this complex was tested.

Cells were cultivated for 4 h with a concentration of COSAN-TATE corresponding to 100ppm of boron. Then, they were digested in a 1:1 mixture of sulphuric and nitric acids for 60 min at more than 100°C. Next, Triton x-100 (at 5%) was added as well as an internal standard solution of Y and Sr and the boron concentration was measured by Inductively coupled plasma optical emission spectroscopy (ICP-OES). Prior to the measurements, a calibration curve was made with the standard solution and known quantities of boron.

3 samples of tumour cell lines were tested. The results are shown in the table below:

| **Tumor cell line** | **Boron uptake (COSAN-TATE)** |
|---|---|
| Sample 1 | 26,37 ppm |
| Sample 2 | 37,21 ppm |
| Sample 3 | 29,94 ppm |

These data demonstrate that boron was effectively and similarly taken up by three different cell lines after incubation with COSAN-TATE.

### Example 6. Comparative test of use of COSAN-TATE and Boronphenylalanine (BPA) for cancer therapy by boron neutron capture therapy.

A comparative test of the effect of neutron irradiation was carried out. For this test, sample 1 of the tumour cell line used in example 5 (bronchial carcinoid cell line) was treated with COSAN-TATE (example 2) and compared with the same cell line treated with the currently used compound boronphenylalanine (BPA). The cells treated with both compounds were able to irradiate with a neutron beam cell treated under conditions of equivalent radiation dose delivered. For comparison, the uptake of BPA in these tumour cells was found to be 16.89 ppm.

Cells were cultured in complete medium at 70% confluence in quartz cuvettes 24 hours before neutron irradiation. Two samples were prepared, one with COSAN-TATE and other with BPA, and they were added to the culture media 4h prior to irradiation. The medium was replaced with fresh complete medium before irradiating the cells for different times to achieve different neutron doses.

After irradiation, cells were detached and then prepared for clonogenic assays. Cells were seeded in triplicate on six-well plates at appropriate numbers, based on the irradiation dose, to form colonies of more than 50 cells in around two weeks. Colonies were fixed with 90% ethanol, stained with crystal violet, and then counted. The survival was expressed according to the plating efficiency calculated.

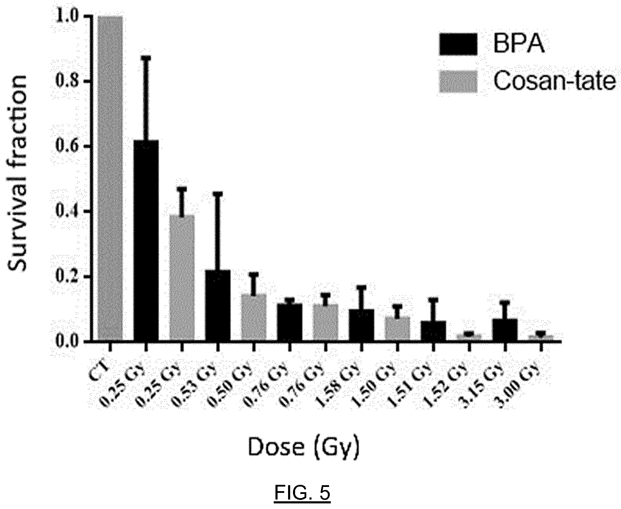

Figure 5 shows that the killing effect of tumor cells is higher for COSAN-TATE sample than BPA sample, suggesting it improves the therapy in patients treated with the peptide-bound boron cluster complex of the present invention.

### REFERENCES

[1] Jyothi, V. G. S., & Kommineni, N. (2024). Peptide Conjugated Boron Neutron Capture Therapy for Enhanced Tumor Targeting. Nanotheranostics, 8(4), 458.
[2] Schirrmacher, E., Schirrmacher, R., Beck, C., Mier, W., Trautman, N., & Rösch, F. (2003). Synthesis of a Tyr3-octreotate conjugated closo-carborane [HC2B10H10]: a potential compound for boron neutron capture therapy. Tetrahedron letters, 44(51), 9143-9145.
[3] Betzel, T., Heß, T., Waser, B., Reubi, J. C., & Roesch, F. (2008). Closo-Borane conjugated regulatory peptides retain high biological affinity: synthesis of closo-borane conjugated Tyr3-octreotate derivatives for BNCT. Bioconjugate chemistry, 19(9), 1796-1802.
[4] Cabrera-González, J. (2016). Nuevos derivados basados en clústeres de boro: desarrollo, aproximaciones fotofísicas y funcionalización de plataformas. Universitat Autònoma de Barcelona.
[5] Kawai, K., Nishimura, K., Okada, S., Sato, S., Suzuki, M., Takata, T., & Nakamura, H. (2020). Cyclic RGD-functionalized closo-dodecaborate albumin conjugates as integrin targeting boron carriers for neutron capture therapy. Molecular Pharmaceutics, 17(10), 3740-3747.
[6] Mishiro, K., Imai, S., Ematsu, Y., Hirose, K., Fuchigami, T., Munekane, M., ... & Ogawa, K. (2022). RGD peptide-conjugated dodecaborate with the Ga-DOTA complex: a preliminary study for the development of theranostic agents for boron neutron capture therapy and its companion diagnostics. Journal of Medicinal Chemistry, 65(24), 16741-16753.
[7] Parejo, L., Chaari, M., Santiago, S., Guirado, G., Teixidor, F., Núñez, R., & Hernando, J. (2021). Reversibly Switchable Fluorescent Molecular Systems Based on Metallacarborane-Perylenediimide Conjugates. Chemistry-A European Journal, 27(1), 270-280.

## Claims

1. A peptide-bound boron cluster complex **characterized in that** it has a chemical structure according to one of the following formulas: 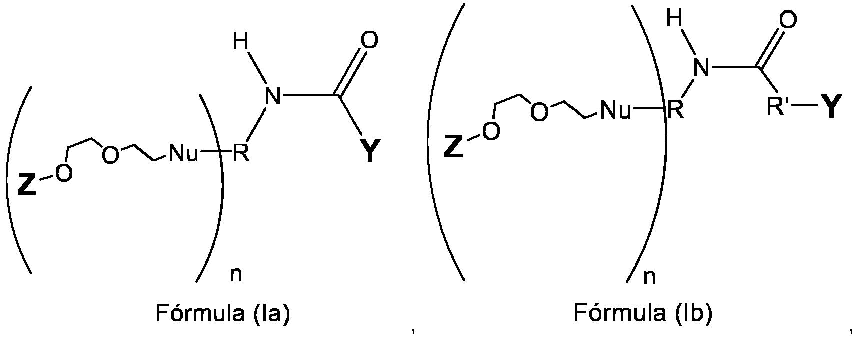 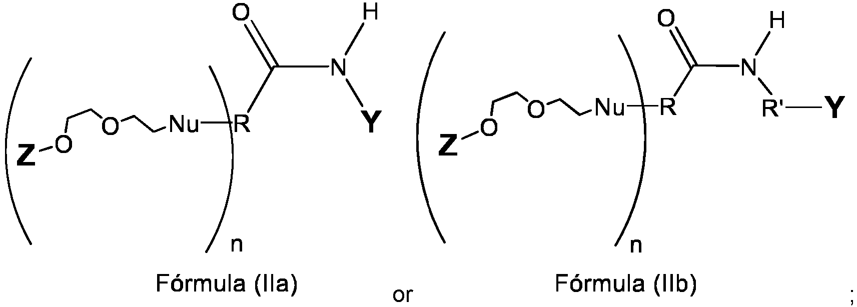 wherein
Z is an icosahedral boron compound selected from the group consisting of closo-dodecaborate, carboranes or isomers thereof, or metallacarboranes;
Nu is a nucleophile selected from the group consisting of halides, thioethers, amines, alkoxides, and carboxylates or derivatives thereof;
R is an aliphatic organic chain, an aromatic organic chain or a polyether chain;
R' is a peptide formed with α-amino acids, β-amino acids, γ-amino acids and/or molecules with at least one -COO⁻ group and/or with at least one -NH₂ group linked together by peptide bonds;
Y is a peptide selected from the group consisting of somatostatin analog peptide, prostate-specific membrane antigen (PSMA) ligand, neuropeptide Y, arginylglycylaspartic acid (RGD) peptides, GE11 peptide, angiopep-2 peptide, gastrin-releasing peptide receptor (GRPR) analogs, luteinizing-hormone-releasing hormone (LHRH) analogue, pseudo-neurotensin derivatives or Lyp-1 peptide, chlorotoxine and apicoline analog; or
Y is an anti-solid tumor antibody or a fragment thereof;
n is between 1 and 8;
and its pharmaceutically acceptable salts, solvates hydrates or mixtures thereof.

2. The peptide-bound boron cluster complex according to claim 1, wherein the icosahedral boron compound is selected from the group consisting of *closo* [B₁₂H₁₂]²⁻, *closo* [CB₁₁H₁₂]⁻, isomers of *closo* C₂B₁₀H₁₂ carboranes (*ortho-*(1,2), *meta*-(1,7), and *para*-(1,12)), isomers of *nido* [C₂B₁₀H₁₁]⁻ carboranes (*ortho*-(1,2) and *meta*-(1,7)) and metallacarboranes [3,3'-M(1,2-C₂B₉H₁₁)₂]⁻, wherein M is a metal selected from Co (III) and Fe(III).

3. The peptide-bound boron cluster complex according to anyone of claims 1 to 2, wherein Y is a peptide selected from the group consisting of Tyr3-octreotate, Tyr3-octreotide, (S)-2-(3-((S)-5-Amino-1-carboxypentyl)ureido)pentanedioic Acid, neuropeptide Y, RGD peptide, cyclic RGD peptide, iRGD peptide, GE11 peptide, angiopep-2 peptide, GRPR analogs, [D-Lys6]-GnRH-I, [Lys8-9]NT(8-13) or Lyp-1 peptide.

4. The peptide-bound boron cluster complex according to anyone of claims 1 to 2, wherein Y is an anti-solid tumor antibody selected from the group consisting of anti-nectin-4, anti-EGFR and anti-TROP2.

5. The peptide-bound boron cluster complex according to claim 1, wherein the complex has the following chemical structure:

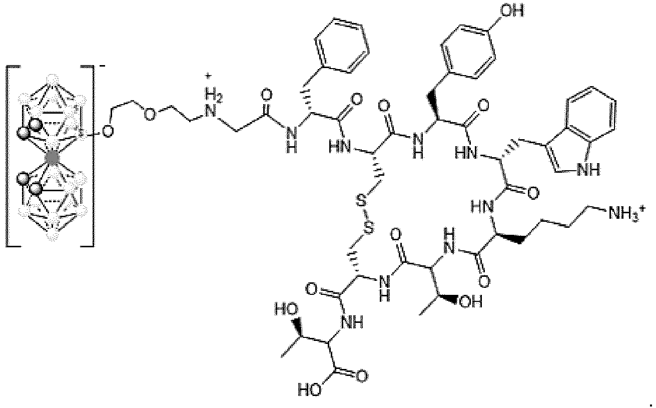

6. The peptide-bound boron cluster complex according to claim 1, wherein the complex has the following chemical structure;

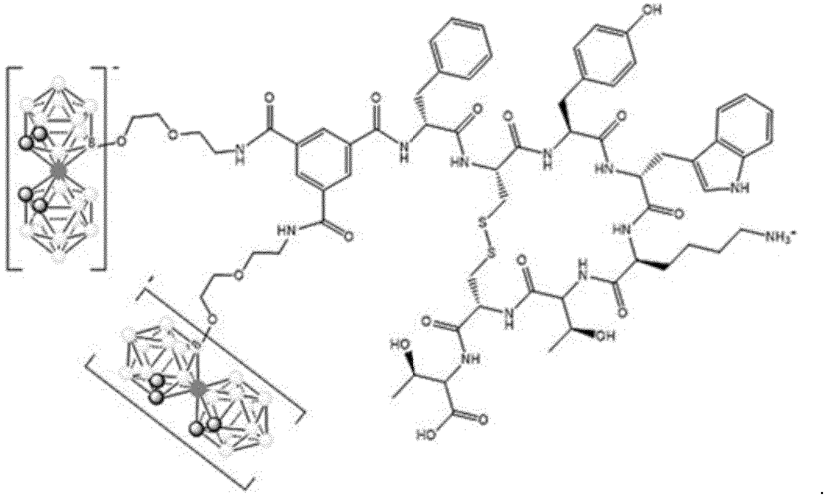

7. The peptide-bound boron cluster complex according to claim 1, wherein the complex has the following chemical structure:

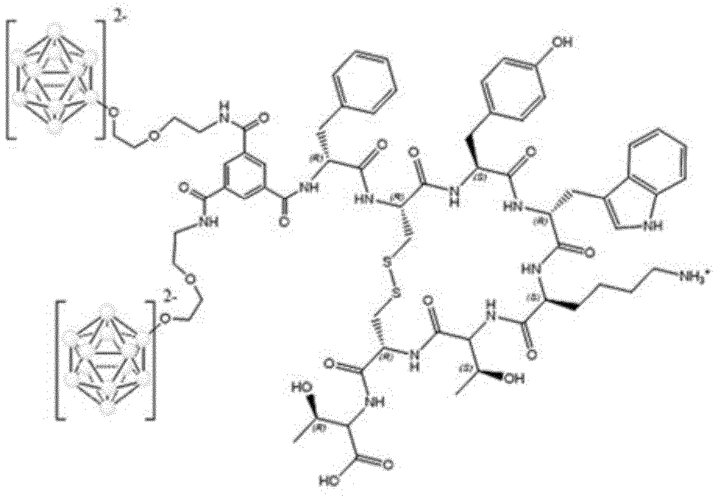

8. The peptide-bound boron cluster complex according to claim 1, wherein the complex has the following chemical structure:

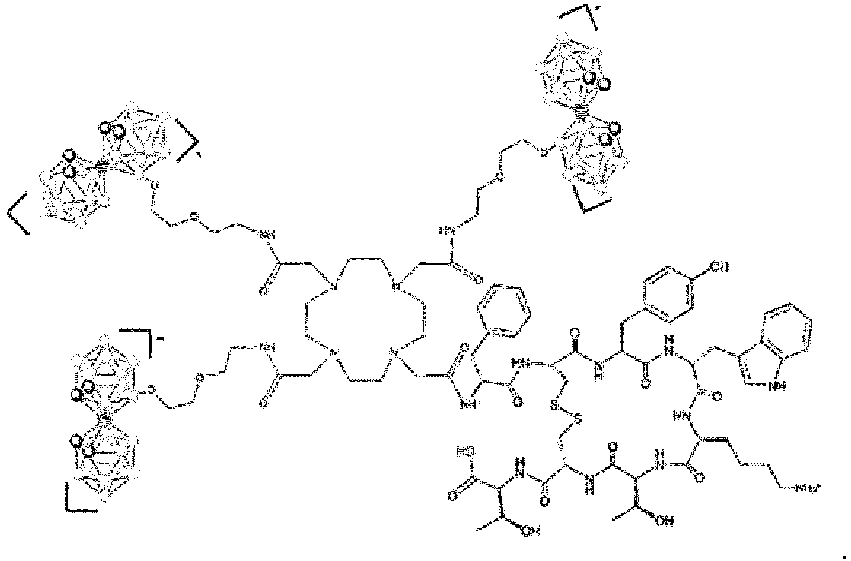

9. The peptide-bound boron cluster complex according to anyone of claims 1 to 8, wherein R has at least one macrocyclic chelator attached for coordination with a radioisotope.

10. The peptide-bound boron cluster complex according to claim 9, wherein the radioisotope is selected from the group consisting of technetium-99m, gallium-68, lutetium-177, terbium isotopes or an atom such as gadolinium.

11. The peptide-bound boron cluster complex according to anyone of claims 1 to 8, wherein at least one B-H vertex of the icosahedral boron compound is replaced by B-X wherein X is a halogen.

12. A pharmaceutical composition comprising the peptide-bound boron cluster complex according to anyone of claims 1 to 11 and a pharmaceutically acceptable excipient.

13. The peptide-bound boron cluster complex according to anyone of claims 1 to 11 or the pharmaceutical formulation according to claim 12, for use in cancer therapy by boron neutron capture therapy.
